# FASCICULE DE BREVET EUROPEEN

(11) **EP 1 368 002 B1**
(45) Date de publication et mention de la délivrance du brevet: **17.05.2006**
(21) Numéro de dépôt: 02702462.9
(22) Date de dépôt: 07.02.2002
(51) Int. Cl.: A61K 9/20, A61K 31/517

(54) **PROCEDE DE FABRICATION D'UN COMPRIME FLOTTANT INCLUANT DE L'ALFUZOSINE**
VERFAHREN ZUR HERSTELLUNG EINER SCHWIMMFÄHIGEN TABLETTE MIT ALFUZOSIN
METHOD FOR PRODUCING A FLOATING TABLET CONTAINING ALFUZOSINE

(30) Priorité: 08.02.2001 FR 0101711; 21.12.2001 FR 0116705
(43) Date de publication de la demande: 10.12.2003
(73) Titulaire: Ellipse Pharmaceuticals, 33600 Pessac (FR)
(72) Inventeur: BORDES, Frédérique, 33200 Bordeaux (FR); CUART, Sylvie, F-33600 Pessac (FR); TERRASSIN, Laurent, 33110 Le Bouscat (FR)
(74) Mandataire: Fantin, Laurent
(86) Numéro de dépôt international: PCT/FR2002/000474
(87) Numéro de publication internationale: WO 2002/062321

(56) Documents cités:
- WO-A-94/27582
- FR-A- 2 304 354
- FR-A- 2 784 583
- US-A- 6 149 940

## Description

La présente invention concerne un procédé de fabrication d'un comprimé flottant incluant en tant que principe actif de l'alfuzosine. L'invention couvre aussi le comprimé obtenu.

On sait qu'il est utile pour certains principes actifs de prévoir une libération contrôlée afin de limiter le nombre de prises tout en assurant une efficacité optimale. En effet, l'administration de formes orales à libération immédiate nécessite plusieurs administrations quotidiennes qui, en plus de l'inconfort engendré peuvent perturber l'observance du traitement.

En ce qui concerne plus particulièrement le chlorhydrate d'alfuzosine, utilisé notamment dans le traitement des hypertrophies bénignes de la prostate et dans le traitement de l'éjaculation précoce, la libération contrôlée est encore plus nécessaire.

Cette molécule à demi-vie d'élimination courte présente la caractéristique d'être absorbée préférentiellement dans la partie haute du tractus gastro-intestinal, plus particulièrement dans le duodénum et le jéjunum.

Dans le brevet US-A- 6 149 940, on a proposé un comprimé destiné à la voie orale visant à libérer du chlorhydrate d'alfuzosine. Ce comprimé est constitué de deux couches au moins qui comprennent :
- la première, un mélange de polymères hydrophiles, 5,00 à 90,00 %,et
- la deuxième, un mélange de polymères hydrophiles et de chlorhydrate d'alfuzosine. Cette deuxième couche est comprimée de façon convenable et comprend des substances auxiliaires pour que le chlorhydrate d'alfuzosine soit libéré dans un intervalle de temps prédéterminé.

Il est prévu de contrôler cette libération par adjonction d'une troisième couche constituée également de polymères hydrophiles en sorte de protéger la couche contenant le chlorhydrate d'alfuzosine et de ralentir la libération, au moins au départ.

Un tel comprimé présente des inconvénients. En effet., le comprimé est initialement plus lourd que la densité des fluides gastriques dans lesquels il se trouve plongé. Un risque est celui de voir le comprimé entraîné avant d'avoir acquis un pouvoir de flottaison suffisant. La cinétique de gonflement des polymères hydrophiles est lente, de l'ordre d'une demi-heure, et ce délai est suffisant pour qu'il y ait évacuation. Le gonflement suffisant qui éviterait le passage à travers le pylore est atteint plusieurs heures après l'ingestion si bien que les caractéristiques dimensionnelles et l'éventuelle retenue mécanique ne peuvent pallier le problème de la non flottaison initiale.

Le séjour gastrique dépend aussi de l'anatomie de l'individu, taille du pylore, rythmes d'ouverture et de fermeture, si bien que toutes ces considérations permettent de constater de fortes variations.

En conclusion, on peut difficilement disposer d'une libération contrôlée dans de telles conditions.

La demande de brevet français N° 2 784 583 décrit un comprimé multicouches, perfectionné en ce sens qu'il comporte un couple effervescent qui génère du dioxyde de carbone au contact des fluides gastriques. Ces bulles de dioxyde de carbone confèrent des capacités de flottaison à ce comprimé qui lui permettent de séjourner dans l'estomac de façon moins aléatoire.

Cette flottaison est complétée par la présence de polymères hydrophiles qui gonflent et augmentent le volume du comprimé au cours du temps.

Néanmoins, un tel comprimé reste complexe à produire industriellement car il faut travailler en atmosphère à humidité contrôlée à cause du couple effervescent. Cela engendre aussi plus de précautions pour stocker les produits obtenus compte tenu de la présence de ce couple effervescent, si bien que la fabrication est d'un coût plus élevé.

Le couple effervescent utilisé contient du sodium dont l'apport n'est pas souhaitable notamment dans le cas d'un régime hyposodé, ce qui est un autre inconvénient.

Le procédé de fabrication selon la présente invention, d'un comprimé contenant de l'alfuzosine permet de garantir un temps de séjour optimal dans l'estomac de l'individu par une flottaison immédiate, une libération contrôlée suivant un profil dont la cinétique peut varier et être adaptée, notamment, entre une cinétique d'ordre 0 ou 1, et de compléter la retenue par flottaison grâce à un gonflement.

De plus, le comprimé obtenu par le procédé selon la présente invention peut être fabriqué à des coûts réduits et ne nécessite pas de conditions délicates de conservation.

A cet effet, le procédé de fabrication selon l'invention, d'un comprimé contenant de l'alfuzosine, se caractérise en ce qu'il comprend les étapes suivantes :
- préparation d'une quantité d'alfuzosine donnée en fonction de la posologie pour une durée de dissolution donnée,
- mélange homogène de cette quantité de principe actif avec une quantité d'excipient de 50,00 à 99,90 % de la masse totale, l'excipient étant choisi parmi au moins un composé de la famille des dérivés cellulosiques et/ou des dérivés de povidone et/ou des dérivés d'acétate de polyvinyle, et
- compression de ce mélange avec une force permettant d'obtenir une pastille monolithique, homogène, à flottaison immédiate dans le milieu gastrique.
   De façon préférentielle, l'excipient est présent à raison de 70,00 à 99,00 % de la masse totale du comprimé.
   L'alfuzosine est sous forme de chlorhydrate d'alfuzosine.
   La compression est une compression directe.
   Pour améliorer l'homogénéité du mélange et la compressibilité du grain, la compression est précédée d'une granulation par voie humide ou sèche.
   Pour obtenir un ordre compris entre 0 et 1 sur la durée de dissolution donnée, on ajoute des modulateurs de cinétique de libération du chlorhydrate d'alfuzosine.
   Comme composé de la famille des dérivés cellulosiques, on retient plus particulièrement l'hydroxypropylméthylcellulose et après granulation, on ajoute cet hydroxypropylméthylcellulose en phase externe.
   Le chlorhyldrate d'alfuzosine est introduit à raison de 1 à 100 mg et plus particulièrement 2,5 ; 5 ou 10 mg.
   Le comprimé a un poids compris entre 50 et 800 mg, préférentiellement entre 200 et 400 mg.
   L'invention couvre aussi le comprimé monolithique obtenu comprenant du chlorhydrate d'alfuzosine.
   Le procédé de fabrication selon la présente invention est décrit ci-après et le comprimé obtenu se trouve simultanément caractérisé.
- La figure 1 représente le tableau de mise en flottaison d'un comprimé obtenu par le procédé, avec des variations de la force de compression en trois lots (A-C).
- La figure 2 représente une courbe de dissolution obtenue avec le comprimé du lot A des compositions du tableau de la figure 1,
- La figure 3 est un tableau de mise en flottaison d'un comprimé avec intégration de lactose, en deux lots (D,E).
- La figure 4 montre deux courbes de dissolution où la cinétique de libération est modifiée.
- La figure 5 montre quatre courbes de dissolution obtenues avec les compositions F à I intégrant comme excipient de base un dérivé d'acétate de polyvinyle.

Selon l'invention, le procédé de fabrication d'un comprimé à base d'atfuzosine et pour les exemples qui vont suivre de chlorhydrate d'alfuzosine, consiste à comprimer directement de la poudre ou à comprimer des granulés obtenus par granulation humide ou sèche. Le mélange de chlorhydrate d'alfuzosine et d'un composé de faible densité permet de réaliser une pastille monolithique, homogène, telle que la densité de ce comprimé soit inférieure à celle des fluides gastriques.

La force de compression doit être adaptée pour respecter la densité de la pastille monolithique, homogène, qui doit rester inférieure à celle des fluides gastriques.

### Exemple 1 :

On prépare un mélange de chlorhydrate d'alfuzosine de 10 mg et d'un dérivé d'hydroxypropylméthylcellulose ou HPMC. On peut utiliser comme dérivé de l'hydroxypropylméthylcellulose, le produit du commerce connu sous la dénomination Métolose 90SH, de viscosité 4 000 centipoises. Le mélange est comprimé directement. La pastille présente un poids final de 400 mg.

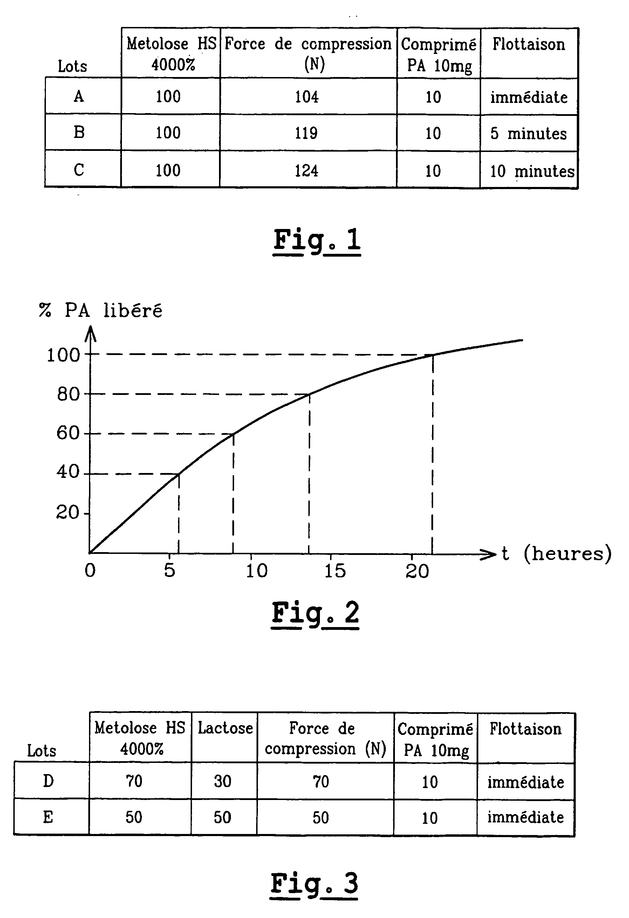

Le tableau de la figure 1 montre que la flottaison est immédiate pour la composition comprimée à 104 N.

On constate que si la force de compression est modifiée avec une augmentation de la force exercée, la densité devient trop importante pour assurer une flottaison immédiate. Il convient donc de maintenir la force de compression de la pastille, en fonction de sa composition, à des valeurs telles que le comprimé flotte immédiatement. Des tests simples en laboratoire permettent de déterminer les valeurs utiles dans chaque cas.

Sur la figure 2, on trouve le profil de dissolution de chlorhydrate d'alfuzosine (pourcentage de Principe Actif libéré en fonction du temps t en heures) dans les conditions habituelles de test de la pharmacopée européenne à savoir appareil à pales, 37°C, à 100 tours/minute et dans un milieu acide à pH2, le dosage étant obtenu par mesure spectrophotométrique à 254 nm.

On constate une cinétique de dissolution qui diminue avec le temps, correspondant sensiblement à une cinétique d'ordre 1 sur 12 à 24 heures, ce qui peut être recherché.

### Exemple 2 :

On substitue à une fraction de dérivé d'hydroxypropylméthylcellulose (Métolose 90SH, 4 000 centipoises), un agent désintégrant à savoir du lactose.

La compression est une compression directe. Les valeurs sont déterminées par des tests connus en laboratoire.

Le tableau de la figure 3 montre que la flottaison est immédiate pour les forces de compression appliquées 50 à 70 N, que le lactose soit présent à 30 ou 50 %.

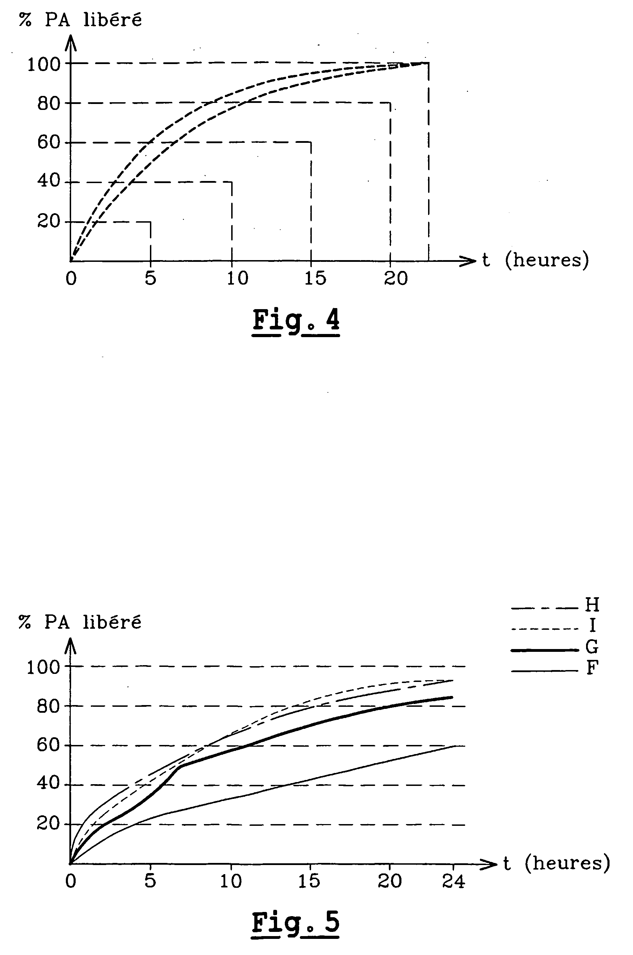

Sur la courbe de la figure 4 (pourcentage de Principe Actif libéré en fonction du temps t en heures), on note de façon naturelle que plus il y a d'agent désintégrant et moins la cinétique de dissolution est linéaire.

Il convient d'ajuster la quantité de lactose en fonction du profil de dissolution recherché.

### Exemple 3 :

On réalise un comprimé comme dans l'exemple 1 mais en choisissant un autre dérivé de l' hydroxypropylméthylcellulose. On prépare un mélange de chlorhydrate d'alfuzosine de 10 mg et on utilise comme dérivé celui commercialisé sous la même dénomination Métolose 90SH, mais avec une viscosité de 15 000 centipoises. La pastille présente un poids final identique de 400 mg. La force de compression est de 82 N. La compression est une compression directe déterminée à partir de tests connus de laboratoire.

La flottaison est immédiate.

La courbe de dissolution n'est pas affectée par le choix de la viscosité.

### Exemple 4 :

Dans cet essai, on reprend le comprimé de l'exemple 1 mais le poids total du comprimé est limité à 200 mg, avec la même force de compression directe.

On constate à nouveau que le comprimé présente une flottaison immédiate.

La courbe de dissolution n'est pas affectée.

### Exemple 5 :

Préalablement à sa compression, le principe actif est mis en suspension dans un solvant approprié et granulé par un polymère retard appartenant à la famille des polymétacrylates (notamment celui commercialisé sous la dénomination Eudragit RS 30 D)

Le principe actif est séché puis passé au travers d'une grille de 0,8 mm.

On ajoute en phase externe un dérivé d' hydroxypropylméthylcellulose et le mélange ainsi obtenu est comprimé. On obtient une flottaison immédiate.

Le profil de libération montre un ralentissement sur 24 heures comme indiqué sur la figure 2. On pourrait ainsi, en ralentissant la cinétique, tendre vers une vitesse de libération constante, c'est-à-dire vers un profil plus linéaire.

### Exemple 6 :

Un essai est maintenant réalisé en ayant recours à un dérivé d'acétate de polyvinyle commercialisé sous la dénomination Kollidon SR. Ce dérivé du commerce comprend de l'ordre de 80% d'acétate de polyvinyle et 19% de povidone et 1% de stabilisants.

Avec cet excipient, la compression directe est facilitée et le recours à un diluant n'est pas nécessaire.

Dans ce cas, les quatre compositions suivantes sont testées :
- F :100% de Kollidon SR
- G: 90% de Kollidon SR et 10% de lactose (agent désintégrant),
- H : 80% de Kollidon SR et 20%-de lactose (agent désintégrant),
- I : 70% de Kollidon SR et 30% de lactose (agent désintégrant).
ce qui conduit aux courbes de la figure 5 qui montrent que le profil peut être ajusté afin d'obtenir une cinétique d'ordre 0 ou 1 sur 12 à 24 heures.

### Exemple 7 :

Un essai est ensuite réalisé avec une étape de granulation préalable à la compression.

On prépare une solution de granulation en milieu aqueux dans laquelle est dissous le principe actif, dans cet exemple, à hauteur de 20%.

On réalise un mélange de 90% de Kollidon SR et de 10% d'un amidon pré-gélatinisé commercialisé sous la dénomination Sepistab ST200.

On réalise l'étape de granulation par pulvérisation de la solution de mouillage décrite ci-avant. Le grain est ensuite séché dans un lit d'air fluidisé jusqu'à l'obtention d'une teneur en eau inférieure à 2%. Le grain est ensuite calibré et la lubrification est réalisée en phase externe avant compression.

On obtient des comprimés dont les cinétiques de dissolution sont sensiblement identiques à celles des exemples 1 et 2.

Par contre, l'étape de granulation permet d'améliorer l'homogénéité du mélange et la compressibilité du grain.

On constate que le procédé permet d'obtenir un comprimé avec une pastille monolithique et homogène, à flottaison immédiate et avec une libération du principe actif sensiblement régulière.

Le dosage du principe actif peut être compris entre 1 et 100 mg et plus particulièrement de 2,5 ; 5 et 10 mg.

Le poids du comprimé obtenu est compris de façon préférentielle entre 50 et 800 mg, plus particulièrement entre 200 et 400 mg.

L'hydroxypropylméthylcellulose ou ses dérivés et/ou les dérivés de povidone et d'acétate de polyvinyle sont présents à raison de 50,00 à 99,00 % de la masse totale du comprimé.

Un agent désintégrant peut être ajouté à raison de 1 à 50% en poids.

D'autres excipients peuvent être ajoutés pour moduler la cinétique de libération comme des argiles, de la polyvinylpyrrolidone réticulée, ou certains dérivés cellulosiques en faibles proportions comme l'hydroxypropylcellulose, la carbométhylcellulose réticulée, ou encore des composés de la famille des N-acétylglucosamine notamment le chitosan.

Des exemples d'agents permettant d'accélérer la cinétique de dissolution sont les agents hydrophiles ajoutés en phase externe : mannitol, lactose, amidon et amidon modifié, sorbitol, xylitol, ou cellulose microcristalline.

Des exemples d'agents diluants liposolubles permettent au contraire de ralentir la libération du principe actif comme : les palmitates, l'huile de ricin, le monostéarate de glycérol. Ces agents contribuent à rendre le comprimé obtenu encore plus flottant par une diminution de la densité du système.

On peut aussi ralentir la cinétique de libération en assurant une granulation du principe actif avec des agents retard comme la polyvinylpyrrolidone, la gélatine, l'éthylcellulose, les dérivés des polyméthncrylntes , l'acide alginique et ses sels.

Les grains ainsi obtenus sont ensuite mélangés à l'hydroxypropylméthylcellulose, ajoutée en phase externe.

Pour la mise en oeuvre des étapes du procédé, il peut être nécessaire d'adjoindre lors de la compression, des lubrifiants tels que : monosténrate de glycérol, les dérivés du polyoxyéthylène glycol, le béhénate de glycérol ainsi que des agents d'écoulement comme les dérivés de la silice colloïdale.

On comprend que le procédé simplifié de fabrication permet de réduire les coûts de façon importante. Non seulement les étapes sont moins nombreuses mais les conditions d'exécution sont moins complexes, du fait notamment de l'absence de couple effervescent.

De plus, la cinétique de dissolution du chlorhydrate d'alfuzosine peut être réglée au moins entre un ordre 0 et 1.

On remarque aussi un effet complémentaire engendré par la nature des constituants utilisés, c'est le gonflement. C'est-à-dire que les capacités de flottaison immédiate sont renforcées au cours du temps car le poids diminue et le volume augmente. De plus, les risques de déstratification des couches des comprimés existants sont supprimés car le caractère monolithique engendre une bonne tenue mécanique.

On constate que la proportion d'excipient est très importante par rapport à celle du principe actif. Il n'était pas évident d'imaginer qu'un tel ration puisse conduire à une flottaison immédiate et qu'il puisse conduire à des maîtrises des profils de libération comme cela vient d'être indiqué ci-avant.

## Revendications

1. Procédé de fabrication d'un comprimé contenant de l'alfuzosine, **caractérisé en ce qu'**il comprend les étapes suivantes :
- préparation d'une quantité donnée d'alfuzosine en fonction de la posologie pour une durée de dissolution donnée,
- mélange homogène de cette quantité de principe actif avec une quantité d'excipient de 50,00 à 99,90 % de la masse totale, l'excipient étant choisi parmi au moins un composé de la famille des dérivés cellulosiques et/ou des dérivés de povidone et/ou des dérivés d'acétate de polyvinyle, et
- compression de ce mélange avec une force permettant d'obtenir une pastille monolithique, homogène, à flottaison immédiate dans le milieu gastrique.

2. Procédé de fabrication selon la revendication 1, **caractérisé en ce que** l'on ajoute de façon préférentielle l'excipient à raison de 70,00 à 99,00 % de la masse totale du comprimé.

3. Procédé de fabrication selon la revendication 1 ou 2, **caractérisé en ce que** la compression exercée est une compression directe après mélange du principe actif avec les excipients.

4. Procédé de fabrication selon la revendication 1 ou 2, **caractérisé en ce que**, préalablement à la compression, on réalise une granulation par voie humide ou sèche.

5. Procédé de fabrication selon l'une quelconque des revendications précédentes, **caractérisé en ce que** l'alfuzosine est sous forme de chlorhydrate d'alfuzosine.

6. Procédé de fabrication selon l'une quelconque des revendications précédentes, **caractérisé en ce que** l'on ajoute des modulateurs de cinétique de libération du chlorhydrate d'alfuzosine pour obtenir un ordre compris entre 0 et 1 sur la durée de dissolution donnée.

7. Procédé de fabrication selon l'une quelconque des revendications précédentes, **caractérisé en ce que** l'on ajoute des modulateurs de cinétique de libération comme les argiles, la polyvinylpyrrolidone réticulée, des dérivés cellulosiques en faibles proportions l'hydroxypropylcellulose, ou la carbométhylcellulose réticulée ou encore des composés de la famille des N-acetylglucosamine notamment le chitosan.

8. Procédé de fabrication selon l'une quelconque des revendications précédentes, **caractérisé en ce que** l'on ajoute un ou plusieurs agents accélérateurs de libération, hydrophiles choisis parmi le mannitol, le lactose, l'amidon ou l'amidon modifié, le sorbitol, le xylitol ou la cellulose microcristalline.

9. Procédé de fabrication selon l'une quelconque des revendications 1 à 7,
**caractérisé en ce que** l'on ajoute un ou plusieurs agents ralentisseurs de libération avec diminution de la densité, choisis parmi les palmitates, l'huile de ricin, le monostéarate de glycérol.

10. Procédé de fabrication selon l'une quelconque des revendications précédentes, **caractérisé en ce que** l'on assure une granulation du principe actif avec des agents retard choisis parmi la polyvinylpyrrolidone, la gélatine, l'éthylcellulose, les dérivés des polyméthacrylates, l'acide alginique et ses sels.

11. Procédé de fabrication selon l'une quelconque des revendications précédentes, **caractérisé en ce que** l'on utilise comme composés de la famille des dérivés cellulosiques l'hydroxypropylméthylcellulose.

12. Procédé de fabrication selon la revendication 10 et 11, **caractérisé en ce que**, après granulation, on ajoute l' hydroxypropylméthylcellulose en phase externe.

13. Procédé de fabrication selon l'une quelconque des revendications précédentes, **caractérisé en ce que** l'on ajoute, lors de la compression, au moins un lubrifiant choisi parmi le monostéarate de glycérol, les dérivés du polyoxyéthylène glycol, le béhénate de glycérol et/ou un agent d'écoulement comme les dérivés de la silice colloïdale.

14. Procédé de fabrication selon l'une quelconque des revendications précédentes, **caractérisé en ce que** le chlorhyldrate d'alfuzosine est introduit à raison de 1 à 100 mg et plus particulièrement 2,5 ; 5 ou 10 mg.

15. Procédé de fabrication selon l'une quelconque des revendications précédentes, **caractérisé en ce que** l'on prépare un comprimé dont le poids est compris entre 50 et 800 mg, préférentiellement entre 200 et 400 mg.

16. Comprimé comprenant du chlorhydrate d'alfuzosine obtenu par la mise en oeuvre du procédé selon l'une quelconque des revendications 1 à 13.

## Claims

1. A method of manufacturing a tablet containing alfuzosin, **characterised in that** it comprises the following steps:
- preparation of a given quantity of alfuzosin according to the dosage for a given dissolution duration,
- homogeneous mixing of said quantity of active principle with a quantity of excipient from 50.00 to 99.90% of the total mass, the excipient being chosen from amongst at least one compound in the family of cellulose derivatives and/or derivatives of povidone and/or derivatives of polyvinyl acetate, and
- the compression of this mixture with a force making it possible to obtain a homogeneous monolithic tablet, with immediate flotation in the gastric environment.

2. A manufacturing method according to claim 1, **characterised in that** the excipient is preferentially added at a rate of 70.00 to 99.00% of the total mass of the tablet.

3. A manufacturing method according to claim 1 or 2, **characterised in that** the compression exerted is a direct compression after mixing of the active principle with the excipients.

4. A manufacturing method according to claim 1 or 2, **characterised in that**, prior to the compression, a granulation is carried out by wet or dry method.

5. A manufacturing method according to any one of the preceding claims, **characterised in that** the alfuzosin is in the form of alfuzosin hydrochlorate.

6. A manufacturing method according to any one of the preceding claims, **characterised in that** modulators for kinetic release of the alfuzosin hydrochlorate are added in order to obtain an order between 0 and 1 on the given dissolution duration.

7. A manufacturing method according to any one of the preceding claims, **characterised in that** modulators for kinetic release are added, such as clays, crosslinked polyvinylpyrrolidone, cellulose derivatives in small proportions, hydroxypropyl cellulose, or crosslinked carbomethylcellulose or compounds in the family of N-acetylglucosamine, in particular chitosan.

8. A manufacturing method according to any one of the preceding claims, **characterised in that** one or more hydrophilic release accelerating agents are added, chosen from among mannitol, lactose, starch or modified starch, sorbitol, xylitol or microcrystallised cellulose.

9. A manufacturing method according to any one of the claims 1 to 7, **characterised in that** there are added one or more release retarding agents with reduction in density, chosen from amongst palmitates, castor oil and glycerol monostearate.

10. A manufacturing method according to any one of the preceding claims, **characterised in that** a granulation of the active principle is provided with retarding agents chosen from among polyvinylpyrrolidone, gelatine, ethyl cellulose, derivatives of polymethacrylates, alginic acid and salts thereof.

11. A manufacturing method according to any one of the preceding claims, **characterised in that** hydroxypropyl methyl cellulose is used as a compound from the family of cellulose derivatives.

12. A manufacturing method according to claim 10 or 11, **characterised in that**, after granulation, hydroxypropyl methyl cellulose is added in external phase.

13. A manufacturing method according to any one of the preceding claims, **characterised in that** there is added, at the time of compression, at least one lubricant chosen from among glycerol monostearate, derivatives of polyoxyethylene glycol, glycerol behenate and/or a flow agent such as derivatives of colloidal silica.

14. A manufacturing method according to any one of the preceding claims, **characterised in that** the alfuzosin hydrochlorate is introduced at an amount of 1 to 100 mg and more particularly 2.5, 5 or 10 mg.

15. A manufacturing method according to any one of the preceding claims, **characterised in that** a tablet is prepared whose weight is between 50 and 800 mg, preferably between 200 and 400 mg.

16. A tablet comprising alfuzosin hydrochlorate obtained by implementation of the method according to any one of claims 1 to 13.

## Patentansprüche

1. Verfahren zur Herstellung einer Alfuzosin enthaltenden Tablette,
**dadurch gekennzeichnet, dass** es die folgenden Schritte umfasst:
- Bereitstellen einer in Abhängigkeit von der Dosierung für eine gegebene Auflösungszeit vorgegebenen Menge an Alfuzosin,
- homogenes Mischen dieser Menge an Wirkstoff mit einer Menge eines Trägerstoffes, die 50,00 bis 99,90 % des Gesamtgewichts beträgt, wobei der Trägerstoff ausgewählt ist aus mindestens einer Verbindung aus der Gruppe der Cellulosederivate und/oder der Povidon-Derivate und/oder der Polyvinylacetat-Derivate, und
- Komprimieren dieser Mischung mit einer Kraft, die groß genug ist, um eine im Magenmilieu sofort aufschwimmende, homogene, monolithische Pastille zu erhalten.

2. Herstellungsverfahren nach Anspruch 1, **dadurch gekennzeichnet, dass** der Trägerstoff vorzugsweise in einem Anteil von 70,00 bis 99,00 % bezogen auf das Gesamtgewicht der Tablette zugesetzt wird.

3. Herstellungsverfahren nach Anspruch 1 oder 2, **dadurch gekennzeichnet, dass** das Komprimieren direkt nach dem Vermischen des Wirkstoffs und der Trägerstoffe erfolgt.

4. Herstellungsverfahren nach Anspruch 1 oder 2, **dadurch gekennzeichnet, dass** vor dem Komprimieren eine feuchte oder trockene Granulation durchgeführt wird.

5. Herstellungsverfahren nach einem der vorangegangenen Ansprüche, **dadurch gekennzeichnet, dass** das Alfuzosin in Form von Alfuzosin-Chlorhydrat vorliegt.

6. Herstellungsverfahren nach einem der vorangegangenen Ansprüche, **dadurch gekennzeichnet, dass** Modulatoren der Kinetik der Freisetzung des Alfuzosin-Chlorhydrats zugesetzt werden, so dass über die vorgegebene Dauer der Auflösung eine Größenordnung zwischen 0 und 1 erzielt wird.

7. Herstellungsverfahren nach einem der vorangegangenen Ansprüche, **dadurch gekennzeichnet, dass** Modulatoren der Kinetik der Freisetzung wie zum Beispiel Ton, vernetztes Polyvinylpyrrolidon, Cellulosederivate in kleinen Mengen, Hydroxypropylcellulose oder vernetzte Carboxymethylcellulose oder aber Verbindungen aus der Gruppe der N-Acetylglucosamine, insbesondere Chitosan, zugefügt werden.

8. Herstellungsverfahren nach einem der vorangegangenen Ansprüche, **dadurch gekennzeichnet, dass** ein oder mehrere hydrophile, die Freisetzung beschleunigende Stoffe, ausgewählt aus der Gruppe umfassend Mannitol, Lactose, Stärke oder modifizierte Stärke, Sorbitol, Xylitol oder mikrokristalline Cellulose, zugesetzt werden.

9. Herstellungsverfahren nach einem der Ansprüche 1 bis 7, **dadurch gekennzeichnet, dass** ein oder mehrere die Dichte herabsetzende und die Freisetzung hemmende Stoffe, ausgewählt aus der Gruppe umfassend die Palmitate, Rizinusöl, Glycerolmonostearat, zugesetzt werden.

10. Herstellungsverfahren nach einem der vorangegangenen Ansprüche, **dadurch gekennzeichnet, dass** eine Granulation des Wirkstoffs durch Retardstoffe, ausgewählt aus der Gruppe umfassend Polyvinylpyrrolidon, Gelatine, Ethylcellulose, Polymethacrylatderivate, Alginsäure und deren Salze, gewährleistet wird.

11. Herstellungsverfahren nach einem der vorangegangenen Ansprüche, **dadurch gekennzeichnet, dass** als Verbindung aus der Gruppe der Cellulosederivate Hydroxypropylmethylcellulose verwendet wird.

12. Herstellungsverfahren nach Anspruch 10 und 11, **dadurch gekennzeichnet, dass** nach der Granulation Hydroxypropylmethylcellulose in der äußeren Phase zugesetzt wird.

13. Herstellungsverfahren nach einem der vorangegangenen Ansprüche, **dadurch gekennzeichnet, dass** während des Komprimierens mindestens ein Gleitmittel, ausgewählt aus der Gruppe umfassend Glycerolmonostearat, Polyoxyethylenglykol-Derivate, Glycerolbehenat, und/oder ein Fließmittel wie die Derivate der kolloidalen Kieselsäure zugesetzt wird.

14. Herstellungsverfahren nach einem der vorangegangenen Ansprüche, **dadurch gekennzeichnet, dass** 1 bis 100 mg, insbesondere 2,5; 5 bzw. 10 mg Alfuzosin-Chlorhydrat eingegeben wird.

15. Herstellungsverfahren nach einem der vorangegangenen Ansprüche, **dadurch gekennzeichnet, dass** eine Tablette bereitet wird, deren Gewicht zwischen 50 und 800 mg, vorzugsweise zwischen 200 und 400 mg beträgt.

16. Tablette, enthaltend Alfuzosin-Chlorhydrat und gewonnen anhand des Verfahrens nach einem der Ansprüche 1 bis 13.
